# EUROPEAN PATENT APPLICATION

(11) **EP 1 329 439 A1**
(43) Date of publication of application: **23.07.2003**
(21) Application number: 03447010.4
(22) Date of filing: 14.01.2003
(51) Int. Cl.: C04B 35/634, C04B 38/00, B22F 1/00

(54) **Method for producing metallic and ceramic foams**

(30) Priority: 26.04.2002 EP 02447076; 14.01.2002 EP 02447006
(71) Applicant: "VLAAMSE INSTELLING VOOR TECHNOLOGISCH ONDERZOEK", afgekort "V.I.T.O.", 2400 Mol (BE)
(72) Inventor: Cooymans, Jozef, 2400 Mol (BE); De Wilde, Anne-Marie, 2400 Mol (BE); Thijs, Ivo, 2400 Mol (BE); Mullens, Steven, 3740 Bilzen (BE); Snijkers, Frans, 3930 Hamont-Achel (BE); Luyten, Jan, 3294 Diest (BE)
(74) Representative: Van Malderen, Joelle

(57) **Abstract**

The present invention is related to a method for producing metallic or ceramic products, comprising the steps of:
- Providing a suitable ceramic or metallic slurry,
- Casting said slurry into a predefined form,
- Drying, optionally calcining and sintering said form,
characterised in that said suitable ceramic or metallic slurry comprises a biogel former and the ceramic or metallic product is shaped as a foam structure.

## Description

### Field of the invention

The present invention is related to a method for producing metallic and ceramic products. More particularly, the present invention is related to the use of natural gel forming compounds in a method for producing metallic or ceramic products, advantageously for the production of foams.

### State of the art

Ceramic products can be manufactured by conventional (dry) techniques on one hand, like pressing (uni-axial or hot or cold isostatical) or, on the other hand, by more advanced (wet) techniques. Such techniques that use a suspension are tape casting and slurry coating. The latter processing route requires that the ceramic powder is part of a suspension in an intermediate stage of the process. Further processing usually consists of drying, calcining and sintering. In addition, machining of the 'green' or the sintered product is possible.

Various production routes for ceramic and metallic foam structures exist. The main characteristics like pore size distribution, relative density, cell morphology and strut density determine the ultimate properties of the foam and thus the field of application. Due to the wide range of application for such materials, several manufacturing routes have been proposed. The major fabrication route is the polyurethane replica technique, where reticulated polyurethane foam is coated with ceramic slurry.

However, the major difficulty with the above mentioned technique exists in a strict control of both the macro- and microstructure of the foam, accompanied with sufficient mechanical strength. For example, the polyurethane replica technique provides foam structures with a well-controlled pore size distribution and strut thickness (owing to a strict process control for the basic material, namely the polyurethane foam). However, as the polymeric foam is calcinated, the strut becomes hollow, giving rise to poor mechanical strength. Other techniques lead to dense struts and strong porous material, but the control over the macro- and microstructure is much more difficult.

As the simultaneous existence of both requirements is essential for the bulk of the applications for ceramic or metallic porous materials, the developments are focussing on new and improved manufacturing routes.

Metallic and ceramic foams are porous materials with very low density (5 to 30% of theoretical density) which are deployed in diverse applications such as filters for molten metals, oven or furnace walls, soot filters, carriers for catalysts, biomedical implants, electro-ceramics, ... . Useful implementations of metallic or ceramic foams requires foams with sufficient strength, which have a controlled structure on both micro and macro level.

Several methods for making such metallic or ceramic foams have been described, however none of these methods provide the superior properties needed for some applications.

### Aims of the invention

The present invention aims to provide a new method for producing metallic and ceramic products and in particular for providing a new method for producing metallic and ceramic foams. A further aim of the invention is to provide a method for producing strong, closed and/or open metallic and ceramic foams. The new method should provide a precise control on the main characteristics of the product, together with good mechanical strength. Further, the present invention aims at avoiding toxicity and pollution issues as present in the current state of the art methods.

### General Description of the invention

The present invention concerns a method for producing metallic or ceramic products, comprising the steps of:
- Providing a suitable ceramic or metallic slurry,
- Casting said slurry into a predefined form,
- Drying, optionally calcining and sintering said form,
characterised in that said suitable ceramic or metallic slurry comprises a biogel former, particularly for slurry coating of organic polymer foams.

Said biogel former is preferably selected from the group consisting of gelatine, ovalbumin, agar, carageenan, inulin, pectine, starch, potato dextrin, guar, caseinate, gellan, alginate, locust bean gum, xanthan and carboxy-methyl-cellulose. Said casting step is preferably foam casting.

The ceramic or metallic product is shaped as a foam structure. The step of providing a suitable ceramic or metallic slurry preferably comprises the steps of:
- Preparation of a stable ceramic or metallic suspension with a suitable dispersant;
- Preparation of a solution of biogel former; and
- mixing the ceramic or metallic suspension with the solution of biogel former.

Preferably, the biogel former is agar. The solid loading of the suspension lies advantageously between 50 and 90 weight%, more advantageously between 60 and 80 weight%.

The method according to this embodiment of the present invention preferably further comprises after the step of casting a gelation step. In a preferred embodiment, the gelation step comprises a temperature change of the slurry to a temperature suitable for gelation of the biogel former.

Another aspect of the present invention is a green ceramic or metallic product obtainable by any of the methods of the present invention and comprising a biogel former. A green ceramic or metallic product is an intermediate product (before calcination and sintering).

### Short description of the figures

Figure 1 shows a flowchart of the method for making ceramic foams with the aid of biogel formers according to the present invention.

Figure 2 shows the influence of (a) the concentration of agar and (b) the concentration of Al₂O₃ on the viscosity of the mixture.

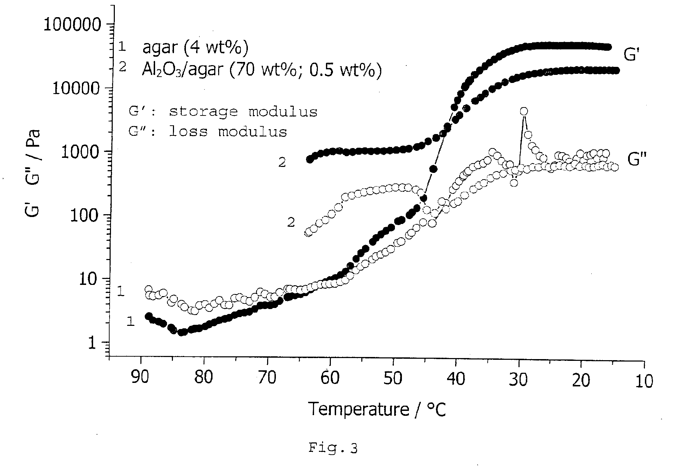

Figure 3 shows the evolution of the viscosity of a solution of agar and a mixture ceramic suspension-agar solution during cooling. The gelation of agar can be observed by the sharp increase of the storage modulus around 40 °C.

Figure 4 depicts the distribution of the cell diameter of a Al₂O₃ porous structure as determined by image analysis (fig. 4 a), together with an optical microscope picture of a 2D slice of the same foam (fig. 4 b).

### Detailed description of the invention

The present invention concerns the use of natural compounds such as biogel formers (also called hydrocolloid or biobinder) as a binder. Preferably, water-based suspensions are used for this, as the natural compounds can readily dissolve in water. Typical examples of such biogel forming compounds are gelatin, ovalbumin, agar, carageenan, ... .

In the present invention, a manufacturing method for making ceramic foams with the aid of biogel formers is described.

In the present invention, a manufacturing method for making ceramic foams with the aid of biogel formers is described.

As important properties of ceramic porous structures like pore size distribution, strut thickness and pore morphology can vary substantially according to the different fabrication routes, these materials are applicable in a wide field. They are used as filters, dust collectors, membranes, catalyst carriers, thermal barriers, lightweight components, bone replacements... . As ceramic foams which are produced with the proposed gel casting procedure using environmentally friendly biogel formers, have greater mechanical strength compared to e.g. the reticulated foam structures with comparable porosity, the applications can even be expanded to situations in which the mechanical strength of the material plays an important role. More specifically, these porous structures could be optimised for their use in the filtration of molten aluminium or other molten metals, as strength and thermal shock resistance are in this application of great importance.

In diesel particulate traps, the characteristics of the ceramic foam must lead to low pressure drop in the filter and at the same time a high trapping efficiency. Accumulated soot can be oxidised using an oxidation catalyst. The ceramic porous structure is in that case used as catalyst support. Mechanical strength is also in this application of great importance.

Several porous structures are already used for bone replacements. A strict control of pore size distribution, porosity and mechanical strength of the foam are essential to guarantee a safe and osteoinductive implant that can either temporarily or life lasting substitute the original bone.

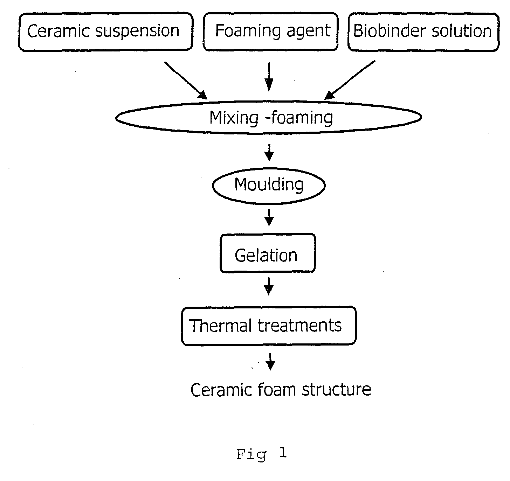

The flow chart of this production method is designed for the production of porous structures with the aid of biogel formers (see fig.1). This manufacturing route makes it feasible to produce ceramic and metallic foams in an environmentally and economically beneficial manner.

Various biogel former systems can be employed in the fabrication of foam structure by gel casting. As naturally occurring gel formers are used as an alternative for the chemical gel formers, major alterations to the conventional gel casting procedure are requisite/inevitable.

The general method can be described as follows:

1. preparation of a stable ceramic suspension, with a suitable dispersant. The solid loading of this suspension can be varied, depending on the desired properties of the foam structure. Preferably, the solid loading is between 60 and 80 weight percentage.

2. preparation of a solution of biogel former. The concentration of this solution can be varied within a specific range (typically preferably between 3 and 7 weight percentage). The experimental conditions are related to the specific biogel former system that will be applied. For example, the use of agar implies heating the mixture above 90 °C. Only in this way, complete dissolution of the agar molecules can be guaranteed. As the gelation takes place at temperatures below 45 °C, this solution must be kept a higher temperature, preventing gelation outside the mould. Alternative systems (based on carrageenan, locust bean gum, xanthan gum, ... or blends of this kind of substances) require other temperatures for handling (dissolution, gelation,...).

3. mixing the ceramic suspension and the biogel former solution. The experimental conditions (like e.g. temperature, other additives) are imposed by the biogel former system used. At this moment, the organic foam agent is added to the mixture. As a result of continuously mixing, a foam structure of ceramic particles in a three dimensional network of biogel former is obtained. The mixing time, the kind of foam agent and the concentration of the foam agent are important experimental parameters as well and contribute to the ultimate foam properties. The mixing time varies preferably between 5 and 10 minutes. During that time, the mixture should be continuously heated. In this way, premature gelation of the biogel former is prevented.

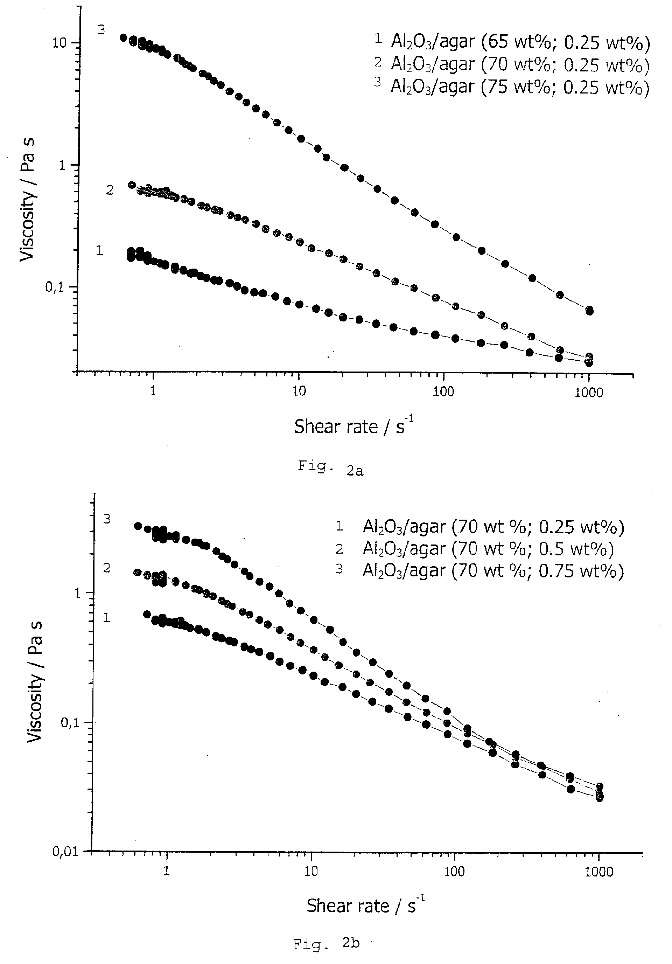

The viscosity of the mixture is determined by both the concentration of ceramic powder and the concentration of biogel former. The mixture ceramic suspension/biogel former solution is characterized by a pseudoplastic behaviour with a viscosity between roughly 10 and 0.1 Pas at γ = 1 s⁻¹). The influence of both the solid loading and the concentration of agar on the viscosity of the mixture is presented in the figure 2. It can be expected that the viscosity of the mixture plays an important role for the ultimate properties of the foam (cell morphology, stability, homogeneity, ...).

Porosity (or foam) in this mixture of ceramic suspension and biobinder solution can be introduced by several methods, amongst which adding foam agents, followed by mixing and blending in such a way that a homogeneous foam structure is obtained. Another method to create porous structure is the introduction of gas or other substances that release gas at these conditions in the mixture.

The created foam is poured into a mould. The concentration of both ceramic suspension and biogel former solution can be varied en will determine in part some characteristics of the foam.

4. The experimental conditions are changed in such a way gelation of the biogel former occurs. Depending on the type of biogel former, the action to allow gelation may vary. For example, when using agar as a biogel former, the foam structure is allowed to cool in the mould in order to achieve gelation of agar (below 40 °C). The cooling step can proceed at normal speed (at room temperature) or can be accelerated by placing the mould in the refrigerator (at about 4°C) or by freeze-drying.

5. the foam is dried, calcinated (at 500-600°C) and subsequently sintered. The exact temperatures depend on the kind of biogel former and of the metallic or ceramic powders used.

The result of this procedure consists of a ceramic foam structure, of which the properties like density (5 to 30 %), pore size distribution and strut thickness are fully controlled. These properties can be changed within certain limits, depending on the interaction between several experimental conditions. Moreover, the mechanical strength of such porous structures is high. Unlike foam structures manufactured by the polyurethane replica technique, the struts of gelcasted ceramic foams are dense. The procedure involves environmentally friendly and relatively cheap starting products. The figure 1 gives a schematic overview of the procedure.

### Examples

### Example 1: mechanical strength of the ceramic foam

A 7 wt% aqueous agar solution is heated to 100 °C in order to assure complete dissolution of the agar molecules. The ceramic suspension consists of 66 wt% Al₂O₃ with Darvan C as a dispersant (1.4 ml/100 g ceramic powder). Before mixing the solutions, the ceramic suspension is ball milled for 30 min. During mixing, the gelation of the agar is prevented by continuously heating the mixture. This mixture consists of 48.7 wt% Al₂O₃ and only 1.7 wt% agar. A foaming agent is added (Tergitol TMN10, Fluka Chemika) and the mixture is stirred for 5 min. The resulting foam structure is allowed to cool in a mould. After drying, calcination and sintering, the ceramic foam structure is cut into shapes. The porosity of this structure has been determined as 85 %. The mechanical strength has been tested by 3-point bending tests. The mean bending strength for this particular foam structure is 3.8 MPa. For comparison, foam structures with comparable porosity and which are manufactured by the replica technique are characterised by bending strength not exceeding 2 MPa.

### Example 2: Cell diameter

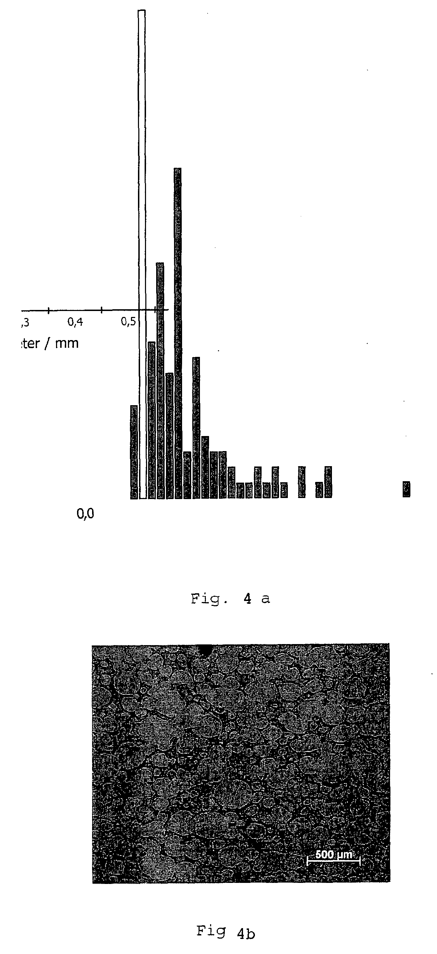

Depending on the different experimental parameters like solids loading, nature and concentration of the foaming agent, mixing time and agar concentration, a variety of foam structures can be obtained each with their characteristic distribution of cell diameters. As an example, a typical cell diameter distribution is presented in figure 4 a, together with a optical microscope picture of an imbedded slice of the foam (figure 4 b).

For the production of this structure, a 50 wt% Al₂O₃-suspension (with Darvan C as dispersant) was heated to 60 °C and mixed with a 4 wt% agar solution that had been heated at 90 °C to assure complete dissolution. After adding a foaming agent (Tergitol TMN10, Fluka Chemika), the mixture is mixed using a conventional mixer for 2 min. The composition of the final mixture consists of 44 wt% Al₂O, 1.8 wt% agar and 0.9 vol/wt% foaming agent. The gelation of the biobinder is accelerated by cooling in the refrigerator for 1 h. Afterwards, the ceramic structure is allowed to dry at room temperature, is calcinated (600°C) and sintered at 1700 °C.

### Example 3: Metallic foam as scaffold for bone replacement

For the production of metallic foam structures, a solution of a gel former, a metallic suspension and a foam agent are mixed. A 5 wt% aqueous agar solution is heated to 90 °C in order to assure complete dissolution of the agar molecules. The metallic suspension consists of 70-90 wt% stainless steel (or other metals like Ti, Ni-Ti, Ta,... ) and 0.1-2 wt % (on water basis) thickener (ammonium- or natrium alginate, methylcellulose, gelatine,...).

During mixing, the mixture is heated above 50 °C in order to prevent the gelation of the agar. This final mixture consists of ∼60 wt% stainless steel powder and only 1-1.5 wt% agar. A foaming agent is added (tergitol TMN10, Fluka Chemika) and the mixture is stirred for 15 min using a conventional mixing system. The resulting foam structure is poured in a mould and allowed to cool. Drying can occur at room temperature and under controlled humidity. The thermal treatments consists of a calcination between 500 °C and 600°C and the sintering at ∼1280 °C under vacuum in order to prevent oxidation of the metallic structure. This procedure results in a metallic foam structure with a density of 5-40 %. The average cell size can be varied depending on different processing parameters between 100 and 500 µm, making these structures ideal porous scaffolds for bone replacement.

## Claims

1. A method for producing metallic or ceramic products, comprising the steps of:
• Providing a suitable ceramic or metallic slurry,
• Casting said slurry into a predefined form,
• Drying, optionally calcining and sintering said form,
**characterised in that** said suitable ceramic or metallic slurry comprises a biogel former and the ceramic or metallic product is shaped as a foam structure.

2. Method as in claim 1, **characterised in that** said biogel former is selected from the group consisting of gelatine, ovalbumin, agar, carageenan, inulin, pectine, starch, potato dextrin, guar, caseinate, gellan, alginate, locust bean gum, xanthan, carboxy-methyl-cellulose.

3. Method as in claim 1 or 2, **characterised in that** said casting is foam casting.

4. The method as in claim 1, wherein the step of providing a suitable ceramic or metallic slurry comprises the steps of:
• Preparation of a stable ceramic or metallic suspension with a suitable dispersant;
• Preparation of a solution of biogel former; and
• mixing the ceramic or metallic suspension with the solution of biogel former.

5. The method as in anz of the claims 1 to 4, wherein the biogel former is agar.

6. The method as in claim 4, wherein the solid loading of the suspension lies between 50 and 90 weight%.

7. The method as in any of the claims 1 to 6, further comprising, after the step of casting, a gelation step.

8. The method as in claim 7 wherein the gelation step comprises a temperature change of the slurry to a temperature suitable for gelation of the biogel former.

9. Green ceramic or metallic product obtainable by the method as in any of the claims 1 to 8 and comprising a biogel former.
